Europäisches Patentamt
European Patent Office
Office européen des brevets

(19) (11) Veröffentlichungsnummer: **0 076 446**
**A2**

# EUROPÄISCHE PATENTANMELDUNG

(12)

(21) Anmeldenummer: 82108911.7

(22) Anmeldetag: 27.09.82

(51) Int. Cl.³: A 01 N 43/64
A 01 N 43/50
//C07D405/06

(30) Priorität: 02.10.81 DE 3139250

(43) Veröffentlichungstag der Anmeldung:
13.04.83 Patentblatt 83/15

(84) Benannte Vertragsstaaten:
BE CH DE FR GB IT LI NL

(71) Anmelder: BASF Aktiengesellschaft
Carl-Bosch-Strasse 38
D-6700 Ludwigshafen(DE)

(72) Erfinder: Rentzea, Costin, Dr.
Neuenheimer Landstrasse 72
D-6900 Heidelberg(DE)

(72) Erfinder: Reissenweber, Gernot, Dr.
Drosselstrasse 15
D-6737 Boehl-Iggelheim(DE)

(72) Erfinder: Feuerherd, Karl-Heinz, Dr.
25-11-Higashi Yukigaya 4-chome
Ohta-Ku(JP)

(72) Erfinder: Jung, Johann, Dr., Dipl.-Landwirt
Hardenburgstrasse 19
D-6703 Limburgerhof(DE)

(54) **Pflanzenwachstumsregulierende Azolylalkyl-2,3-dihydrobenzofurane und ihre Verwendung.**

(57) Die vorliegende Erfindung betrifft Mittel zur Regulierung des Pflanzenwachstums, enthaltend ein Azolylalkyl-2,3-dihydrobenzofurane der Formel

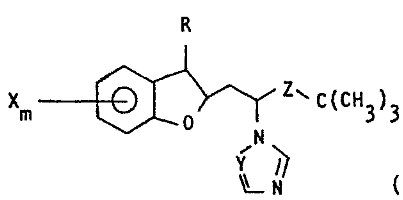

(1)

in der R, X, Y, Z und m die in der Beschreibung angegebene Bedeutung besitzen.

EP 0 076 446 A2

# Pflanzenwachstumsregulierende Azolylalkyl-2,3-dihydrobenzofurane und ihre Verwendung

Die vorliegende Erfindung betrifft Mittel zur Regulierung des Pflanzenwachstums, die Azolylalkyl-2,3-dihydrobenzofurane enthalten, sowie die Verwendung dieser Verbindungen zur Beeinflussung des Pflanzenwachstums.

Es ist bekannt, daß bestimmte 2-Halogen-ethyl-trialkylammonium-halogenide pflanzenwachsregulierende Eigenschaften aufweisen (vgl. US-PS 3 156 554). So läßt sich mit Hilfe von (2-Chlorethyl)-trimethylammoniumchlorid das Pflanzenwachstum beeinflussen. Allerdings ist die Wirksamkeit dieses Stoffes, vor allem bei niedrigen Aufwandmengen, nicht immer ausreichend.

Es ist ferner bekannt, 3,3-Dimethyl-2-(1,2,4-triazolyl-(1))-1-(4-chlorbenzoyl)-butan zur Regulierung des Pflanzenwachstums zu verwenden. (DE-OS 2 739 352).

Es wurde nun gefunden, daß Verbindungen der Formel I

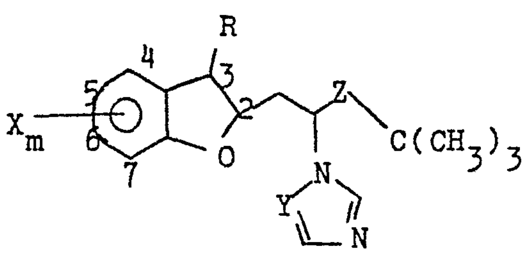 (I),

in der

R Wasserstoff oder Alkyl mit 1 bis 4 C-Atomen bedeutet,

X Wasserstoff, Fluor, Chlor, Brom, Jod, Nitro, Trifluormethyl, Alkyl mit 1 bis 5 C-Atomen, Alkoxy mit 1 bis 3 C-Atomen oder Alkenyl mit 2 bis 3 C-Atomen, Phenyl oder Phenoxy bedeutet und

Mu/P

m eine ganze Zahl von 1 bis 4 bedeutet, wobei die einzelnen Gruppen X gleich oder verschieden sind, wenn m größer als 1 ist,

Y N oder CH bedeutet und

Z eine -CO- oder $-CR^1OR^2$-Gruppe bedeutet, in welcher

$R^1$ Wasserstoff oder $C_1$-$C_4$-Alkyl bedeutet und

$R^2$ Wasserstoff, einen gegebenenfalls durch Chlor substituierten Alkylrest mit 1 bis 6 C-Atomen oder einen gegebenenfalls durch Chlor substituierten Alkenylrest mit 3 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl und Alkyl mit 1 bis 4 C-Atomen substituiert sein kann, oder $R^2$ einen $-CO-R^3$-Rest bedeutet, wobei

$R^3$ einen gegebenenfalls durch Halogen, Alkoxy mit 1 bis 2 C-Atomen, Oxo (=O) oder Carboxyalkyl mit 2 bis 5 C--Atomen substituierten Alkylrest mit 1 bis 4 C-Atomen oder einen aromatischen Rest bedeutet,

sowie deren Säureadditionssalze und Metallkomplexsalze, hervorragend zur Beeinflussung des Pflanzenwachstums geeignet sind und eine sehr gute Pflanzenverträglichkeit besitzen.

Die Verbindungen der Formel I enthalten chirale Zentren und werden im allgemeinen in Form von Racematen oder als Diastereomerengemische erhalten. Die Diastereomeren lassen sich beispielsweise durch Säulenchromatographie trennen oder aufgrund von Löslichkeitsunterschieden in reiner Form isolieren. Aus solchen einheitlichen Diastereomeren kann man mit bekannten Methoden einheitliche Enantiomere erhalten. Für die Anwendung als Wachstumsregulatoren sind sowohl die einheitlichen Diastereomeren bzw. Enantiomeren wie auch deren bei der Synthese anfallenden Gemische geeignet. Bevorzugt werden die letzteren verwendet.

X bedeutet vorzugsweise 5-Fluor, 6-Fluor, 5-Chlor, 6-Chlor, 5-Brom, 6-Brom, 5-Methyl, 7-Methyl, 5-Methoxy, 6-Methoxy, 5-Trifluormethyl, 6-Trifluormethyl, 5-tert.-Butyl, 6-tert.-Butyl, 5-Nitro, 6-Nitro, 4,6-Dichlor, 5,7-Dichlor, 5,6-Dichlor, 5-Brom-7-chlor, 5-Chlor-7-brom, 5-Chlor-7-methyl, 5-Methyl-7-chlor, 4,5,7-Trichlor, 5,7-Dimethyl, 4,6-Dimethyl, 5-Ethoxy, 5-n-Butoxy und 5-Phenoxy.

R und $R^1$ bedeuten vorzugsweise Wasserstoff, Methyl, Ethyl, n-Propyl, Isopropyl und n-Butyl.

$R^2$ bedeutet vorzugsweise Wasserstoff, Methyl, Ethyl, n-Propyl, n-Butyl, 4-Chlorbutyl, n-Pentyl, n-Hexyl, Allyl, 1-Buten-2-yl, Propargyl, Benzyl, 4-Fluorbenzyl, 4-Chlorbenzyl, 4-Brombenzyl, 2,4-Dichlorbenzyl und 4-Trifluormethylbenzyl. $R^3$ bedeutet vorzugsweise Methyl, Ethyl, n-Propyl, Isopropyl, Chlormethyl, Chlorpropyl, Methoxymethyl, Acetonyl ($CH_3COCH_2$), Vinyl und Propenyl.

Geeignete Säureadditionssalze sind beispielsweise die Bromide, Chloride, Sulfate, Nitrate, Phosphate, Oxalate oder Dodecylsulfonate. Die Wirksamkeit der Salze geht auf das Kation zurück, so daß die Wahl des Anions, soweit es pflanzenverträglich ist, beliebig ist. Metallkomplexsalze sind beispielsweise die Komplexe mit Kupfersulfat, Kobaltsulfat, Nickelchlorid oder Eisenchlorid.

Die Verbindungen der Formel I lassen sich herstellen, indem man ein 2,3-Dihydrobenzofuran-2-methylhalogenid der Formel II

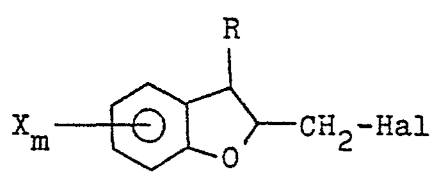 (II)

worin R, X und m die oben angegebenen Bedeutungen haben und Hal Chlor, Brom oder Iod bedeutet, mit einem Keton der Formel III

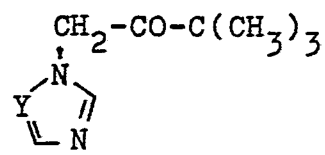

(III)

worin Y die oben genannte Bedeutung hat oder mit dessen Alkalienolat umsetzt und die so erhaltenen Verbindungen gegebenenfalls anschließend mit Grignard-Reagentien versetzt oder reduziert und - falls gewünscht - danach verethert oder verestert.

Das Verfahren zur Herstellung der Ketone der Formel I in denen Z eine CO-Gruppe darstellt, besteht darin, daß man bekannte Ketone der Formel III (DE-OS 26 38 479) oder deren Alkalienolate mit 2,3-Dihydrobenzofuran-2-methylhalogeniden II (s. Toyoshima u. Mitarb., Yakugaku Zasshi. 88, 503 (1968)), gegebenenfalls in Gegenwart einer starken anorganischen oder organischen Base, gegebenenfalls in Gegenwart eines Lösungs- oder Verdünnungsmittels die Ein- oder Zweiphasensysteme bilden und gegebenenfalls unter Zusatz eines Reaktionsbeschleunigers und/oder Phasentransferkatalysators alkyliert, beispielsweise entsprechend der folgenden Umsetzung.

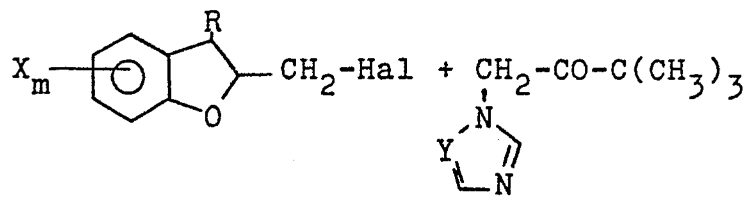

(II) (III)

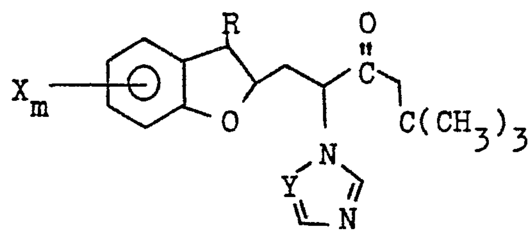

(I, Z = CO)

Hierzu können die Ketone III zunächst zu den Alkalienolaten metalliert werden, indem man sie vorzugsweise in Gegenwart eines polaren aprotischen Lösungsmittels wie Dimethylformamid, Dimethylacetamid, Acetonitril, Sulfolan, Tetrahydrofuran oder Dimethoxyethan mit 1 bis 4 Äquivalenten, bevorzugt 1,0 Äquivalenten Metallierungsreagens wie Natriumhydrid, Lithiumdiisopropylamid, Calciumhydrid oder Kalium-tert.-butoxid bei -10 bis 100°C, vorzugsweise bei 0 bis 70°C umsetzt. Nach anschließender Zugabe von 0,8 bis 2,0, bevorzugt 1,0 Äquivalenten des jeweiligen Benzofuran-1-methylhalogenids II und gegebenenfalls eines Reaktionsbeschleunigers wie Natriumbromid, Natriumiodid, Kaliumbromid, Kaliumiodid oder eines Kronenethers wie 14-Krone-5, 18-Krone-6 oder Dibenzo-18-Krone-6 erhält man bei Reaktionstemperaturen zwischen 0 und 100°C, bevorzugt bei 3 bis 60°C die Ketone der Formel I.

Die Ketone der Formel I (Z = CO) lassen sich gegebenenfalls in Gegenwart eines Lösungs- oder Verdünnungsmittels, bei Temperaturen zwischen -20 und 150°C, bei Normaldruck oder

unter erhöhtem Druck mit Wasserstoff in Gegenwart eines Hydrierungskatalysators, mit Alkalihydrid oder komplexen Bor- bzw. Aluminiumhydriden, mit Aluminiumisopropoxid und Isopropanol, mit Natriumdithionit oder elektrochemisch zu den sekundären Alkoholen der Formel I, in der Z die Gruppe $\underset{OH}{\underset{|}{-CH-}}$ bedeutet, reduzieren.

Geeignete Lösungs- oder Verdünnungsmittel hierfür sind beispielsweise Wasser, Methanol, Ethanol, Isopropanol, Essigsäure, Diethylether, Tetrahydrofuran, Dioxan, Essigester, Toluol, Dimethylformamid oder entsprechende Gemische.

Zur katalytischen Hydrierung verwendet man Platin- oder Palladiumkatalysatoren auf inerten Trägern und hydriert bei Drucken von 20 bis 100 bar bis keine Wasserstoffaufnahme mehr erfolgt.

Für die als Reduktionsmittel verwendbaren Hydride seien beispielsweise Natriumhydrid oder Lithiumaluminiumhydrid genannt.

Die Ketone der Formel I (Z = CO) lassen sich schließlich mit einer Grignardverbindung der Formel

$$R^1MgHal \qquad (IV)$$

in der $R^1$ $C_1$-$C_4$-Alkyl bedeutet und in der Hal Chlor, Brom oder Jod bedeutet, gegebenenfalls in Gegenwart eines Lösungs- oder Verdünnungsmittels und gegebenenfalls in Gegenwart eines Magnesium- oder Tetraalkylammonium-halogenids bei Temperaturen zwischen 0 und 100°C zu den tertiären Alkoholen der Formel I in der Z die Gruppe

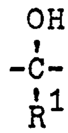

darstellt, alkylieren.

Als Lösungsmittel kommen bevorzugt Ether in Frage, wie Diethylether, Di-n-propylether, Methyl-tert.-butylether, Tetrahydrofuran, Dimethoxyethan oder Anisol, ferner tertiäre Amine wie N,N-Dimethylanilin, 1,2-Bis-(dimethylamino)-ethan sowie Phosphorsäure-tris(dimethylamid); gegebenenfalls kann man die Reaktion auch im Gemisch dieser Lösungsmittel mit aliphatischen oder aromatischen Kohlenwasserstoffen wie n-Pentan, n-Hexan, Cyclohexan, Benzol oder Toluol durchführen. Als ausbeutesteigernde Salze, die die üblichen Nebenreaktionen unterdrücken, kommen insbesondere wasserfreie Magnesiumhalogenide wie wasserfreies Magnesiumbromid oder wasserfreie Tetraalkylammoniumhalogenide wie z.B. Tetra-n-butyl-ammoniumchlorid in Frage. Die Reaktionstemperaturen lassen sich je nach Lösungsmittel zwischen 0 und 100°C variieren, bevorzugt sind Temperaturen zwischen 0 und 60°C. Die hierbei primär entstandenen Magnesiumalkoholate werden anschließend durch Hydrolyse mit verdünnten wäßrigen Säuren, wie Salzsäure, Schwefelsäure oder bevorzugt Essigsäure oder besonders bevorzugt mit wäßriger Ammoniumchloridlösung in die Alkohole übergeführt und diese nach Entfernen der wäßrigen Phase, falls gewünscht, in üblicher Weise durch Extraktion, Umkristallisieren oder Chromatographie gereinigt.

Die so erhaltenen Alkohole der Formel I (Z = -CH-OH- und

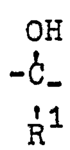 lassen sich mit Alkylierungsmitteln der Formel V

$$R^2 - Hal \qquad (V)$$

worin $R^2$ und Hal die oben angegebenen Bedeutungen haben, gegebenenfalls in Gegenwart eines Lösungs- oder Verdünnungsmittels die Ein- oder Zweiphasensysteme bilden, gegebenenfalls unter Zusatz einer anorganischen Base und gegebenenfalls unter Zusatz eines Reaktionsbeschleunigers und/oder eines Phasentransferkatalysators zu den entsprechenden Verbindungen, in denen Z $-CH-OR^2-$ oder $-R^1C-OR^2-$ bedeutet, verethern.

Hierfür eignen sich beispielsweise folgende Lösungs- oder Verdünnungsmittel: Diethylether, Tetrahydrofuran, Dioxan, n-Pentan, monohalogenierte Kohlenwasserstoffe mit 2 bis 6 C-Atomen wie beispielsweise Chlorethan, Bromethan, 1--Chlorpropan, 1-Brompropan, 1-Chlorbutan, 1-Brombutan, 1-Chlorpentan, 1-Brompentan, 1-Chlorhexan, 1-Bromhexan, ferner Cyclohexan, Methylenchlorid, Chloroform, Toluol, Chlorbenzol, Xylol oder Dimethylformamid.

Als anorganische Basen seien beispielsweise genannt: Alkali- und Erdalkalihydroxide wie Natriumhydroxid, Kaliumhydroxid, Calciumhydroxid, Bariumhydroxid, Alkali- und Erdalkalicarbonate wie Natriumcarbonat, Kaliumcarbonat, Natriumhydrogencarbonat, Kaliumhydrogencarbonat, Calciumcarbonat, Bariumcarbonat, Alkali- oder Erdalkalialkoholate wie Natriummethylat, Natriumethylat, Magensiummethylat, Natriumisopropylat oder Kalium-tert.-butylat.

Geeignete Reaktionsbeschleuniger sind beispielsweise Metallhalogenide wie Natriumbromid, Natriumjodid, Kaliumbromid, Kaliumjodid und Kronenether wie 14-Krone-5, 18--Krone-6, Dibenzo-18-Krone-6 oder Dicyclohexano-18-Krone--6.

Als Phasentransferkatalysatoren kommen vorzugsweise quaternäre Ammoniumsalze wie Tetrabutylammoniumchlorid, -bromid, -iodid oder -hydrogensulfat, Benzyl-triethylammoniumchlorid, Methyl-trioctylammoniumchlorid und -bromid und Phosphoniumsalze wie Tetrabutylphosphoniumbromid und -iodid in Frage.

Die Veresterung der Alkohole der Formel I (Z = CHOH und
 OH
 |
-C-) kann mit Säurechloriden oder -anhydriden der Formeln
 |
 $R^1$
$R^3$-CO-Cl (VI) oder $(R^3\text{-CO})_2O$ (VII) gegebenenfalls in Gegenwart eines Lösungs- oder Verdünnungsmittels, gegebenenfalls unter Zusatz eines säurebindenden Mittels und gegebenenfalls unter Zusatz eines Reaktionsbeschleunigers erfolgen. Hierfür eignen sich dieselben Basen wie für die Veretherung. Zusätzlich können tertiäre Amine wie Trimethylamin, Triethylamin, N,N-Dimethylanilin, N,N-Dimethylcyclohexylamin, N-Methylpiperidin oder Pyridin als Basen und Imidazol, N-Methylimidazol, 1,2,4-Triazol, 4-Dimethylaminopyridin und 4-Pyrrolidinopyridin als Reaktionsbeschleuniger verwendet werden.

Mit geeigneten Basen z.B. Alkalihydrid wie Natriumhydrid oder Alkali- oder Erdalkalialkoholaten wie Natriummethylat können die Alkohole auch in einer ersten Umsetzung zunächst in ihre Alkoholat-Salze überführt werden und dann als solche zur Reaktion gebracht werden.

Die so erhaltenen Verbindungen der Formel I, in denen Z $-CHO-CO-R^3$- oder $-R^1CO-CO-R^3$ bedeutet, werden nach üblichen Methoden isoliert, gegebenenfalls gereinigt und gegebenenfalls mit Säuren oder Metallsalzen zu Salzen bzw. zu Metallkomplexen umgesetzt.

Die Herstellung der Verbindungen der Formel I wird durch folgende Beispiele erläutert.

Beispiel 1

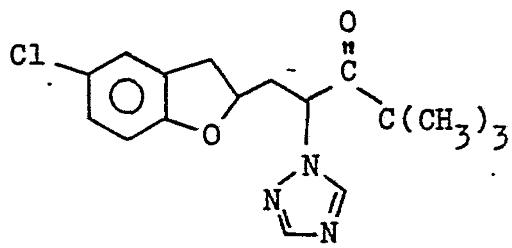

Zu einer gerührten Suspension von 9,6 g (0,4 Mol) Natriumhydrid in 120 ml trockenem Dimethylformamid (DMF) wird die Lösung von 66,7 g (0,4 Mol) 2,2-Dimethyl-4-(1,2,4-triazol-1-yl)-butan-3-on (siehe DE-OS 26 38 470) in 80 ml DMF unter einer trockenen Stickstoffatmosphäre bei 20 bis 25°C zugetropft. Nach dreistündigem Rühren wird bei 25°C die Lösung von 99 g (0,4 Mol) 2,3-Dihydro-2-brommethyl-5-chlorbenzofuran (siehe S. Toshima u. Mitarb., Yakugaku Zasshi., 88, 503 (1968)) in 80 ml DMF zugetropft und das Reaktionsgemisch weitere 48 Stunden nachgerührt. 50 ml Eiswasser werden vorsichtig zugetropft und das Gemisch wird im Vakuum eingeengt. Der Rückstand wird zwischen 500 ml Methylenchlorid und 200 ml Wasser verteilt, die organische Phase zweimal mit je 200 ml Wasser gewaschen, über $Na_2SO_4$ getrocknet und eingeengt. Der Rückstand wird mit 50 ml Petrolether bei 10°C 1 Stunde gerührt und der Niederschlag abgesaugt. Man erhält 55,4 g (41,5 % d.Th.) 1-(2',3'-Dihydro-5'-chlorbenzofuran-2'-yl)-2-(1,2,4-triazol-1-yl)-4,4-dimethylpentan-3-on als weiße Kristalle vom Schmelzpunkt 131 bis 134°C (Wirkstoff Nr. 1).

Beispiel 2

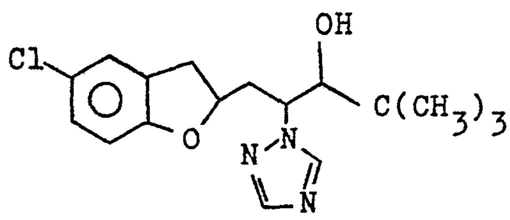

Zu einer Lösung von 35,3 g (0,105 Mol) 1-(2',3'-Dihydro-5'-chlordibenzofuran-2'-yl)-2-(1,2,4-triazol-1-yl)-4,4-dimethylpentan-3-on in 250 ml Methanol werden zwischen 0 und +5°C 4,6 g (0,12 Mol) Natriumborhydrid portionsweise zugegeben. Nach 12-stündigem Rühren bei 20°C wird das Gemisch eingeengt. Der Rückstand wird mit 150 ml 20 %iger (Gew.-%) Kalilauge und mit 400 ml Methylenchlorid 1 Std. gerührt. Die organische Phase wird zweimal mit je 70 ml Wasser gewaschen, über Natriumsulfat getrocknet und eingeengt. Der Rückstand kristallisiert bei 10°C nach Zugabe von 70 ml Petrolether. Man isoliert 33,9 g (96,2 % d.Th.) 1-(2',3'-Dihydro-5'-chlorbenzofuran-2'-yl)-2-(1,2,4-triazol-1-yl)-4,4-dimethylpentan-3-ol als farblose Kristalle vom Schmelzpunkt 63 bis 66°C (Wirkstoff Nr. 2).

Beispiel 3

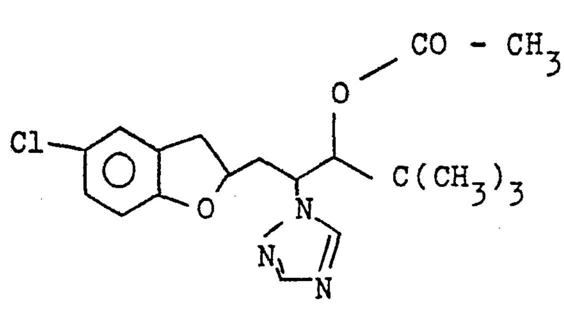

Eine Mischung aus 12,5 g (0,0375 Mol) 1-(2',3'-Dihydro-5'--chlorbenzofuran-2'-yl)-2-(1,2,4-triazol-1-yl)-4,4-dimethylpentan-3-ol, 4,6 g (0,0375 Mol) 4-Dimethylaminopyridin und 100 ml Acetanhydrid werden 12 Stunden bei 60°C gerührt und anschließend im Vakuum eingeengt. Der Rückstand wird mit 300 ml Ether und 100 ml einer 6 %igen Natriumhydrogencarbonatlösung 1 1/2 Stunden gerührt, die organische Phase abgetrennt, über Natriumsulfat getrocknet und eingeengt. Der feste, farblose Rückstand wird mit n-Pentan gerührt und abgesaugt.

Man erhält 10,5 g (74,2 % d.Th.) 1-(2',3'-Dihydro-5'-chlorbenzofuran-2'-yl)-2-(1,2,4-triazol-1-yl)-3-acetoxy-4,4-dimethylpentan vom Schmelzpunkt 98 bis 99°C (Wirkstoff Nr.3).

Beispiel 4

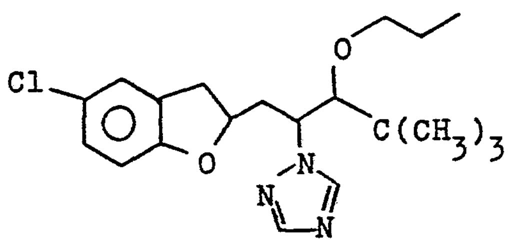

Unter kräftigem Rühren wird eine Mischung aus 12,5 g (0,0375 Mol) 1-(2',3'-Dihydro-5'-chlorbenzofuran-2'-yl)-2--(1,2,4-triazolyl)-4,4-dimethylpentan-3-ol, 100 ml 1-Chlorpropan, 4 g Tetrabutylammoniumhydrogensulfat und 65 g 50 %iger wäßriger Natriumhydroxidlösung 24 Stunden auf 40°C erwärmt. Die Mischung wird sodann mit 300 ml Wasser versetzt und fünfmal mit je 80 ml Methylenchlorid extrahiert. Die vereinigten Extrakte werden fünfmal mit je 100 ml Wasser ausgeschüttelt, über Magnesiumsulfat getrocknet und im Vakuum - schließlich bis 80°C und 0,1 mbar - eingeengt. Der ölige Rückstand wird mit 30 ml Petrolether 3 Tage bei +5°C im Kühlschrank stehen gelassen.

Die ausgefallenen, farblosen Kristalle werden abgesaugt, mit Pentan gewaschen und getrocknet.

Man erhält 9,8 g (69,2 % d.Th.) 1-(2',3'-Dihydro-5'-chlorbenzofuran-2'-yl)-2-(1,2,4-triazol-1-yl)-3-(1-propyloxy)-4,4-dimethylpentan vom Schmelzpunkt 75 bis 78°C (Wirkstoff Nr. 4).

Beispiel 5

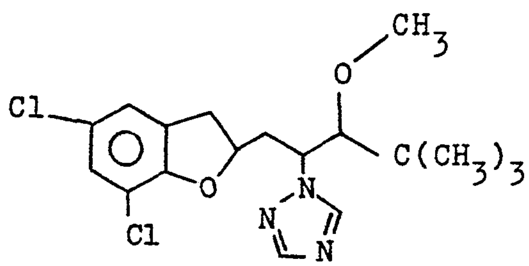

Zu einer Suspension von 2,4 g (0,1 Mol) Natriumhydrid in 100 ml trockenem Tetrahydrofuran wird eine Lösung von 22 g (0,06 Mol) 1-(2',3'-Dihydro-5',7'-dichlorbenzofuran-2'-yl)-2-(1,2,4-triazol-1-yl)-4,4-dimethylpentan-3-ol (Beispiel 17) in 60 ml Tetrahydrofuran getropft. Nach 12-stündigem Rühren bei Raumtemperatur wird das Gemisch mit 14,2 g (0,1 Mol) Iodmethan versetzt. Nach 20stündigem Rühren wird das Reaktionsgemisch vorsichtig mit 40 ml Wasser zersetzt und eingeengt. Der Rückstand wird in 350 ml Ether aufgenommen, zweimal mit je 100 ml Wasser gewaschen, die Etherphase getrocknet und eingeengt. Der Rückstand wird mit 50 ml Petrolether bei 5°C 1 Stunde gerührt. Der kristalline, farblose Niederschlag wird abgesaugt, mit Petrolether gewaschen und getrocknet.

Man erhält 11,4 g (49,5 % d.Th.) 1-(2',3'-Dihydro-5',7'-dichlorbenzofuran-2'-yl)-2-(1,2,4-triazol-1-yl)-3-methoxy-4,4-dimethylpentan vom Schmelzpunkt 117 bis 118°C (Wirkstoff Nr. 5).

Beispiel 6

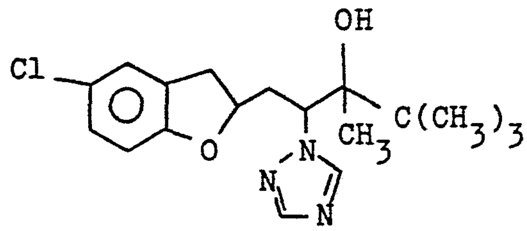

Zu einer Lösung von 5,4 g (0,0406 Mol) Methylmagnesiumbromid in 30 ml Tetrahydrofuran tropft man eine Lösung von 7 g (0,021 Mol) 1-(2',3'-Dihydro-5'-chlorbenzofuran--2'-yl)-4,4-dimethyl-pentan-3-on (Beispiel 1) in 50 ml Tetrahydrofuran und rührt 20 Stunden bei Raumtemperatur. Die Mischung wird sodann in 800 ml 10 %iger wäßriger Ammoniumchloridlösung eingerührt und zweimal mit je 200 ml Dichlormethan extrahiert. Nach dem Waschen der organischen Phase und Trocknen über Magnesiumsulfat wird das Dichlormethan im Vakuum abgedampft. Der Rückstand kristallisiert bei +5°C nach Zugabe von 10 ml Petrolether und 10 ml Ether. Man isoliert 3,2 g (43,6 % d.Th.) 1-(2',3'-Dihydro-5'--chlorbenzofuran-2'-yl)-2-(1,2,4-triazol-1-yl)-3,4,4-trimethylpentan-3-ol als farblose Kristalle vom Schmelzpunkt 144 bis 146°C (Wirkstoff Nr. 6).

Weitere Beispiele für die Verbindungen der Formel (I) sind in der folgenden Tabelle 1 enthalten.

| Beispiel Nummer | $X_m$ | R | Y | Z | Fp. ($^oC$) | IR (Film) [$cm^{-1}$] |
|---|---|---|---|---|---|---|
| 7 | 5-Cl | H | CH | CO | Harz | 3104,2970,1703,1465,1225,<br>1215,1162,1100,802,732. |
| 8 | 5-Cl | H | CH | CHOH | 163-165 | |
| 9 | 5-Cl | $CH_3$ | N | CO | 128-130 | |
| 10 | 5-Cl | $CH_3$ | N | CHOH | Harz | 2955,1492,1464,1265,1220,<br>1128,1008,965,805,672. |
| 11 | $5-CH_3-O$ | H | N | CO | 120-122 | |
| 12 | $5-CH_3$ | H | N | CO | Harz | 3110,2970,1708,1480,1360,<br>1268,1230,1200,1128,802. |
| 13 | $5-CH_3$ | H | N | CHOH | Harz | 3300,2970,1610,1485,1270,<br>1200,1010,982,805,680. |
| 14 | 5-tert.-$C_4H_9$ | H | N | CO | Harz | 3120,2968,1710,1490,1360,<br>1270,1230,1135,818,680. |
| 15 | 5,7-$Cl_2$ | H | N | CO | Harz | 3105,2956,1700,1566,1445,<br>1262,1128,992,850,756. |
| 16 | 5,7-$Cl_2$ | H | N | CO | 155-159 | (Hydrochlorid) |
| 17 | 5,7-$Cl_2$ | H | N | CHOH (Diastereomer I) | 137-139 | |
| 18 | 5,7-$Cl_2$ | H | N | CHOH (Diastereomerengemisch) | Harz | 2950,1458,1270,1192,1130,<br>1008,980,850,755,672. |
| 19 | 5,7-$Cl_2$ | H | N | $-CH-OCO-CH_3$ | 99-101 | |
| 20 | 5,7-$Cl_2$ | H | N | -CH-O 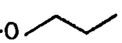 | 95- 97 | |
| 21 | 5,7-$Cl_2$ | H | N | -CH-O 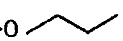 | 111-113 | |
| 22 | 5,7-$Cl_2$ | H | N | $-CH-O-CH_2$ 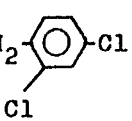 (Diastereomer I) | 146-148 | |

| Beispiel Nummer | $X_m$ | R | Y | Z | Fp. (°C) | IR (Film) [$cm^{-1}$] |
|---|---|---|---|---|---|---|
| 23 | 5,7-$Cl_2$ | H | N | -CH-O-$CH_2$- 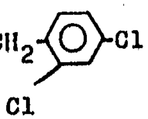 (Diastereomerengemisch) | Harz | 3122,2950,1576,1456,1212, 1122,1068,842,752,672. |
| 24 | 5,7-$Cl_2$ | $CH_3$ | N | CO | Harz | 3118,2960,1708,1445,1262, 1160,1126,962,878,848,750. |
| 25 | 5,7-$Cl_2$ | $CH_3$ | N | CH-OH | Harz | 2960,1495,1450,1268,1190, 1162,1128,1010,960,850,750. |
| 26 | 4,6-$Cl_2$ | H | N | CO | 90- 92 | |
| 27 | 4,6-$Cl_2$ | H | N | CH-OH | 122-124 | |
| 28 | 4,6-$Cl_2$ | H | N | -CH-$OCOCH_3$ | 116-118 | |
| 29 | 4,6-$Cl_2$ | H | N | -CH-O 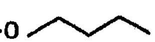 | 92- 94 | |
| 30 | 5-Br, 7-Cl | H | N | CO | Harz | 3120,2970,1711,1495,1454, 1268,1200,1132,854,676. |
| 31 | 5-Br, 7-Cl | H | N | CHOH | Harz | 3350,2960,1496,1454,1270, 1130,1082,980,852,738. |
| 32 | 5-Cl, 7-$CH_3$ | H | N | CO | Harz | 3120,2965,1709,1460,1268, 1190,1130,948,855,676. |
| 33 | 5-Cl, 7-$CH_3$ | H | N | CHOH (Diastereomer I) | 145-147 | |
| 34 | 5-Cl, 7-$CH_3$ | H | N | CHOH (Diastereomerengemisch) | Harz | 3200,2980,1510,1470,1280, 1200,1135,1088,864,788 |
| 35 | H | H | N | CO | Harz | 3120,2965,1708,1588,1470, 1360,1268,1220,1130,748. |
| 36 | H | H | N | CHOH | Harz | 3290,2955,1592,1474,1270, 1225,1130,866,746,675. |

| Beispiel Nummer | $X_m$ | R | Y | Z | Fp. (°C) | IR (Film) [$cm^{-1}$] |
|---|---|---|---|---|---|---|
| 37 | H | H | N | -CH-O 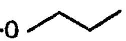 | Harz | 2958,1592,1472,1268,1225, 1128,1090,865,746,672. |
| 38 | H | H | N | -CH-O 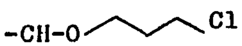 | Harz | 3115,2950,1592,1473,1225, 1130,1096,868,748,675. |
| 390 | H | H | N | -CH-O 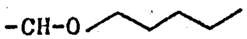 | Harz | 2958,1590,1490,1472,1276, 1225,1130,1090,868,749. |
| 40 | 5-tert.-$C_4H_9$ | H | N | CHOH | 126-128 | |
| 41 | 5-$CH_3O$ | H | N | CHOH | 95- 97 | |

Die neuen Verbindungen der Formel I können praktisch alle Entwicklungsstadien einer Pflanze verschiedenartig beeinflussen und werden deshalb als Wachstumsregulatoren eingesetzt. Für die Wirkungsweise von Pflanzenwachstumsregulatoren gilt nach der bisherigen Erfahrung, daß ein Wirkstoff eine oder auch mehrere verschiedenartige Wirkungen auf Pflanzen ausüben kann.

Die Wirkungsvielfalt der Pflanzenwachstumsregulatoren hängt ab vor allem

a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation),
d) von den geoklimatischen Faktoren, z.B. Sonnenschein, Dauer, Durchschnittstemperatur, Niederschlagsmenge,
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen

weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumshemmern läßt sich beispielsweise bei Winterraps auch die Frostresistenz erheblich erhöhen. Dabei werden einerseits das Längenwachstum und die Entwicklung einer zu üppigen (und dadurch besonders

frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wirkstoffen der Formel I lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel I Ertragssteigerungen durch Eingriffe in den pflanzlichen Stoffwechsel bzw. durch Förderung oder Hemmung des

vegetativen und/oder des generativen Wachstums verusachen.

C. Mit Pflanzenwachstumsregulatoren lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt und Sproßteil der Pflanze ist auch für ein gut kontrollierbares Entblättern der Bäume wesentlich.

Die Wirkung der Verbindungen der Formel I ist besser als bei bekannten Wachstumsregulatoren. Die Wirkung zeigt sich sowohl bei Monokotylen, z.B. Getreide wie Weizen, Gerste, Roggen, Hafer und Reis oder Mais oder Gräsern als auch insbesondere bei Dikotylen (z.B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die Wirkstoffe der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,1 bis 12 kg/ha bevorzugt 0,25 bis 3 kg/ha als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin), Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel oder sonstige oberflächenaktive Mittel, wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind:

I 20 Gewichtsteile der Verbindung des Beispiels 7 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

II 3 Gewichtsteile der Verbindung des Beispiels 29 werden mit 97 Gewichtsteilen feinteiligem Kaolin innig vermischt. Man erhält auf diese Weise ein Stäubemittel, das 3 Gew.% des Wirkstoffs enthält.

III 30 Gewichtsteile der Verbindung des Beispiels 35 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 40 Gewichtsteile der Verbindung des Beispiels 37 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

V 20 Teile der Verbindung des Beispiels 40 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI Man vermischt 90 Gewichtsteile der Verbindung des Beispiels 1 mit 10 Gewichtsteilen N-Methyl-alpha-pyrrolidon und erhält eine Lösung, die zur Anwendung in Form kleinster Tropfen geeignet ist.

VII 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

VIII 20 Gewichtsteile der Verbindung des Beispiels 12 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IX 20 Gewichtsteile der Verbindung des Beispiels 17 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 mol Ethylenoxid an 1 Mol Ricinusöl besteht. Durch Eingießen und feines Verteilen der Lösung in 100.000 Gewichtsteilen Wasser erhält man eine wäßrige Dispersion, die 0,02 Gew.-% des Wirkstoffs enthält.

Die erfindungsgemäßen Mittel können in diesen Aufwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrößerung des Wirkungsspektrums. Bei einer Anzahl solcher Wachstumsregulatormischungen treten auch synergistische Effekte auf, d.h. die Wirksamkeit des Kombinationsproduktes ist größer als die addierten Wirksamkeiten der Einzelkomponenten.

Fungizide, die mit den erfindungsgemäßen Verbindungen kombiniert werden können, sind beispielsweise Dithiocarbamate und deren Derivate, wie
Ferridimethyldithiocarbamat,
Zinkdimethyldithiocarbamat,
Manganethylenbisthiocarbamat,
Mangan-Zink-ethylendiamin-bis-dithiocarbamat,
Zinkethylenbisthiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zinn-(N,N'-propylen-bis-dithiocarbamat) und
N,N'-Polypropylen-bis-(thiocarbamoyl)-disulfid;

Nitrophenolderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazol-acetat,
2,4-Dichlor-6-(o-chloranilino)-s-triazin,
O,O-Diethyl-phthalimidophosphonthionat,
5-Amino-1-(bis-(dimethylamino)-phosphinyl)-3-phenyl-1,2,4-triazol,

5-Ethoxy-3-trichlormethyl-1,2,4-thiadiazol,
2,3-Dicyano-1,4-dithiaanthrachinon,
2-Thio-1,3-dithio-(4,5-b)-chinoxalin,
1-Butylcarbamoyl-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thiol-1-oxid,
8-Hydrochinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-2))-benzimidazol,
Piperazin-1,4-diyl-bis-1-(2,2,2-trichlor-ethyl)-formamid,
2-Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

und verschiedene Fungizide, wie
Dodecylguanidinacetat,
3-(2-(3,5-Dimethyl-2-oxycyclohexyl)-2-hydroxyethyl)-glutarimid,
Hexachlorbenzol,
N-Dichlorfluormethylthio-N,N'-dimethyl-N-phenyl-schwefelsäurediamid,
D,L-Methyl-N-(2,6-dimethyl-phenyl)-N-furyl(2)-alaninat,
D,L-N-(2,6-Dimethyl-phenyl)-N-(2'-methoxyacetyl)-alaninmethylester,
5-Nitro-isophthalsäure-di-isopropylester,
2,5-Dimethyl-furan-3-carbonsäureanilid,
2,5-Dimethyl-furan-3-carbonsäure-cyclohexylamid,
2-Methyl-benzoesäure-anilid,

1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,
1,4-Dichlor-2,5-dimethoxybenzol,
p-Dimethylaminobenzol-diazinnatriumsulfonat,
1-Chlor-2-nitro-propan,

Polychlornitrobenzole, wie Pentachlornitrobenzol, Methylisocyanat, fungizide Antibiotika, wie Griseofulvin oder Kasugamycin, Tetrafluordichloraceton, 1-Phenylthiosemicarbazid, Bordeauxmischung, nickelhaltige Verbindungen und Schwefel.

In den nachfolgenden Beispielen A und B wird die Wirkung der erfindungsgemäß verwendbaren Stoffe als Pflanzenwachstumsregulatoren dargestellt, ohne die Möglichkeit weiterer Anwendungen als Wachstumsregulatoren auszuschließen.

Beispiel A, Gewächshausversuch

Zur Bestimmung der wachstumsregulierenden Eigenschaft der Prüfsubstanzen wurden Testpflanzen auf ausreichend mit Nährstoffen versorgtem Torfkultursubstrat in Kunststoffgefäßen von ca. 12,5 cm Durchmesser angezogen.

Im Vorlaufverfahren wurden die Testsubstanzen in wäßriger Aufbereitung am Tage der Einsaat auf das Saatbeet gegossen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so

gewonnenen Meßwerte wurden zur Wuchshöhe der unbehandelten Pflanzen in Relation gesetzt.

Im Vorlaufverfahren zeigten an Sommergerste beispielsweise die Substanzen der Beispiele 12, 33 eine bessere Wirkung als die Vergleichssubstanz CCC (2-Chlorethyltrimethylammoniumchlorid). Dasselbe gilt im Nachlaufverfahren an Sonnenblumen für die Substanzen der Beispiele 7, 10, 11, 13, 18 bis 20, 25, 28, 31, 33, 34, 37 bis 40, an Soja für die Substanzen der Beispiele 5, 11, 14, 17, 28, 30, 37 bis 40 und an Sommerraps für die Substanzen der Beispiele 2, 3, 5, 9 bis 15, 17, 19, 20, 25 bis 28, 30 bis 35, 37 bis 40.

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Patentansprüche

1. Mittel zur Regulierung des Pflanzenwachstums, enthaltend ein Azolylalkyl-2,3-dihydrobenzofuran der Formel

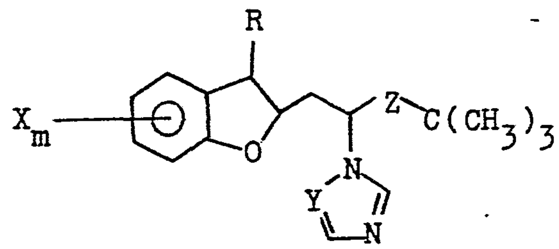

(I)

in der

R Wasserstoff oder Alkyl mit 1 bis 4 C-Atomen bedeutet,

X Wasserstoff, Fluor, Chlor, Brom, Jod, Nitro, Trifluormethyl oder Alkyl mit 1 bis 5 C-Atomen, Alkoxy mit 1 bis 3 C-Atomen oder Alkenyl mit 2 bis 3 C-Atomen, Phenyl oder Phenoxy bedeutet und

m eine ganze Zahl von 1 bis 4 bedeutet, wobei die einzelnen Gruppen X gleich oder verschieden sind, wenn m größer als 1 ist,

Y N oder CH bedeutet und

Z eine -CO- oder $-CR^1OR^2$-Gruppe bedeutet, in welcher

$R^1$ Wasserstoff oder $C_1$-$C_4$-Alkyl bedeutet und

$R^2$ Wasserstoff, einen gegebenenfalls durch Chlor substituierten Alkylrest mit 1 bis 6 C-Atomen oder einen gegebenenfalls durch Chlor substituierten Alkenylrest mit 3 bis 5 C-Atomen oder einen Alkinylrest mit 3 bis 4 C-Atomen oder Benzyl bedeutet, wobei der Benzylrest durch Fluor, Chlor, Brom, Nitro, Trifluormethyl und Alkyl mit 1 bis 4 C-Atomen substituiert sein kann, oder $R^2$ einen $-CO-R^3$-Rest bedeutet, wobei

$R^3$ einen gegebenenfalls durch Halogen, Alkoxy mit 1 bis 2 C-Atomen, Oxo (=O) oder Carboxyalkyl mit 2 bis 5 C-Atomen substituierten Alkyl- oder Alkenylrest mit bis zu 4 C-Atomen oder einen aromatischen Rest bedeutet, sowie deren Säureadditionssalze und Metallkomplexsalze.

2. Mittel zur Regulierung des Pflanzenwachstums gemäß Anspruch 1, dadurch gekennzeichnet, daß sie als Azolylalkyl-2,3-dihydrobenzofuran durch Halogen, $C_1$-$C_3$-Alkoxy oder $C_1$-$C_4$-Alkyl substituierte 1-(2',3'-Dihydrobenzofuran-2'-yl)-2-(1,2,4-triazol-1-yl)-4,4-dimethylpentan-3-one, 1-(2',3'-Dihydrobenzofuran-2'-yl)-2-(1,2,4-triazol-1-yl)-4,4-dimethylpentan-3-ole, 1-(2',3'-Dihydrobenzofuran-2'-yl)-2-(1,2,4-triazol-1-yl)-3-methoxy-4,4-dimethylpentane, 1-(2',3'-Dihydrobenzofuran-2'-yl)-2-(1,2,4-triazol-1-yl)-3-(1-propyloxy)-4,4-dimethylpentane, 1-(2',3'-Dihydrobenzofuran-2'-yl)-2-(1,2,4-triazol-1-yl)-3,4,4-trimethylpentan-3-ole und/oder 1-(2',3'-Dihydrobenzofuran-2'-yl)-2-(1,2,4-triazol-1-yl)-3-methoxy-3,4,4-trimethylpentane enthalten.

3. Mittel zur Regulierung des Pflanzenwachstums, enthaltend eine oder mehrere Verbindungen der Formel I gemäß Anspruch 1 und 2 und einen flüssigen oder festen Trägerstoff sowie gegebenenfalls ein oder mehrere oberflächenaktive Mittel.

4. Verwendung von Verbindungen der Formel I gemäß Anspruch 1 und 2 zur Regulierung des Pflanzenwachstums.

5. Verfahren zur Regulierung des Pflanzenwachstums, dadurch gekennzeichnet, daß man eine oder mehrere Verbindungen der Formel I gemäß Anspruch 1 und 2 auf Pflanzen oder deren Lebensraum einwirken läßt.